(19) Europäisches Patentamt
European Patent Office
Office européen des brevets

(11) Veröffentlichungsnummer: **0 413 223 B1**

# (12) EUROPÄISCHE PATENTSCHRIFT

(45) Veröffentlichungstag der Patentschrift: **10.05.95**

(51) Int. Cl.[6]: **C07D 405/06**, C07D 411/06, C07D 317/72, A01N 43/40, A01N 43/42

(21) Anmeldenummer: **90115046.6**

(22) Anmeldetag: **06.08.90**

Die Akte enthält technische Angaben, die nach dem Eingang der Anmeldung eingereicht wurden und die nicht in dieser Patentschrift enthalten sind.

## (54) N-Heterocyclomethyl-spiroheterocyclen und diese enthaltende Fungizide.

(30) Priorität: **12.08.89 DE 3926769**
**19.12.89 DE 3941870**

(43) Veröffentlichungstag der Anmeldung:
**20.02.91 Patentblatt 91/08**

(45) Bekanntmachung des Hinweises auf die Patenterteilung:
**10.05.95 Patentblatt 95/19**

(84) Benannte Vertragsstaaten:
**AT BE CH DE DK ES FR GB GR IT LI NL SE**

(56) Entgegenhaltungen:
**EP-A- 0 278 352**
**EP-A- 0 281 842**
**EP-A- 0 349 247**
**EP-A- 0 359 979**

(73) Patentinhaber: **BASF Aktiengesellschaft**
**Carl-Bosch-Strasse 38**
**D-67063 Ludwigshafen (DE)**

(72) Erfinder: **Zipplies, Matthias, Dr.**
**Kastanienweg 1**
**D-6945 Hirschberg (DE)**
Erfinder: **Ammerman, Eberhard, Dr.**
**Sachsenstrasse 3**
**D-6700 Ludwigshafen (DE)**
Erfinder: **Lorenz, Gisela, Dr.**
**Erlenweg 13**
**D-6730 Neustadt (DE)**

EP 0 413 223 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft N-Heterocyclomethyl-spiroheterocyclen, diese enthaltende Fungizide und Verfahren zur Bekämpfung von Pilzen mit diesen Verbindungen.

Es ist bekannt, daß Piperidinomethylspiropheterocyclen, wie beispielsweise die Verbindung IX (EP 281 842) fungizide Eigenschaften besitzen.

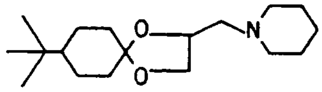

(IX)

Die Wirksamkeit dieser bekannten Verbindung ist jedoch, insbesondere bei niedrigen Aufwandmengen und Konzentrationen, nicht zufriedenstellend.

Es wurde nun gefunden, daß N-Heterocyclomethyl-spiroheterocyclen der allgemeinen Formel I

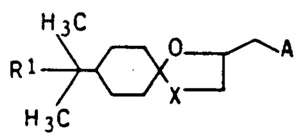

(I)

in welcher

A für die Reste II, III oder IV

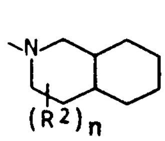

(II)

,

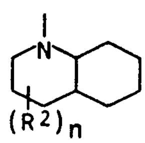

(III)

oder

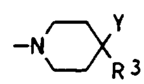

(IV)

$R^2$ für $CH_3$, OH, $OCH_3$,

n für die Zahlen 0 bis 3,

$R^3$ für Hydroxyl, Acyloxy, Alkoxy, gegebenenfalls substituiertes Benzoyloxy, gegebenenfalls substituiertes Benzyloxy, gegebenenfalls substituiertes Alkyl oder gegebenenfalls substituiertes Alkenyl, mit den Substituenten gegebenenfalls substituiertes Aryl, Hydroxyl, Acyloxy, Alkoxy oder Benzyloxy oder

$R^3$ für gegebenenfalls durch Halogen oder Alkyl substituiertes Aryl steht,

X für Sauerstoff,

Y für Wasserstoff oder Hydroxyl,

$R^1$ für Wasserstoff, Alkyl oder für gegebenenfalls substituiertes Cyclohexyl oder gegebenenfalls substituiertes Phenyl steht, sowie deren Säureadditionssalze eine ausgezeichnete fungizide Wirkung gegen phythopathogene Pilze besitzen.

Besonders bevorzugt im Rahmen dieser Erfindung sind N-Heterocyclomethylspiroheterocyclen der allgemeinen Formel I, wobei

A für den Rest II, III oder IV, gemäß Anspruch 1,

n für die Zahlen 0 oder 1,

$R^2$ für $CH_2$, OH, $OCH_3$,

$R^3$ für Hydroxyl, $C_1$-$C_4$-Acyloxy, gegebenenfalls ein- bis dreifach durch Halogen oder $C_1$-$C_4$-Alkyl substituiertes Benzoyloxy oder $C_1$-$C_8$-Alkoxy, gegebenenfalls ein- bis dreifach mit Halogen oder $C_1$-$C_4$-Alkyl substituiertes Benzyloxy,
gegebenenfalls durch OH, Aryl (Phenyl), $C_1$-$C_4$-Acyloxy, Benzoyloxy, $C_1$-$C_8$-Alkoxy, Benzyloxy, substituiertes $C_1$-$C_8$-Alkyl oder gegebenenfalls durch OH, Aryl (Phenyl), $C_1$-$C_4$-Acyloxy, Benzoyloxy, $C_1$-$C_8$-Alkyloxy, Benzyloxy substituiertes $C_3$-$C_4$-Alkenyl oder gegebenenfalls durch 1 bis 3 Halogen- oder $C_1$-$C_4$-Alkylreste substituiertes Phenyl steht

X für Sauerstoff,

Y für Wasserstoff oder Hydroxyl,

$R^1$ für Wasserstoff, für geradkettiges oder verzweigtes Alkyl mit 1 bis 6 Kohlenstoffatomen oder für gegebenenfalls ein- bis dreifach, gleich oder verschieden durch Alkyl mit 1 bis 4 Kohlenstoffatomen oder Halogen substituiertes Phenyl oder gegebenenfalls ein- bis dreifach, gleich oder verschieden durch $C_1$-$C_4$-Alkyl oder Halogen substituiertes Cyclohexyl steht, sowie deren Säureadditionssalze.

Überraschenderweise besitzen die neuen Verbindungen eine bessere fungizide Wirkung als die bekannte Verbindung der Formel IV, welche den neuen Verbindungen ähnlich ist.

Die neuen Verbindungen werden durch die Formel I allgemein definiert.

Darin steht

- A beispielsweise für den trans- bzw. cis-Perhydroisochinolinyl- oder den trans- bzw. cis-Perhydrochinolinyl-Rest bzw. für Gemische der entsprechenden cis-trans-Isomeren oder für den Piperidinylrest,
- $R^2$ für Methyl, Hydroxyl oder Methoxy,
- n beispielsweise für die Zahl 0 oder 1
- $R^3$ beispielsweise für Hydroxyl, Acetoxy, Benzoyloxy, $C_1$-$C_4$-Alkoxy, Methoxy, Ethoxy, iso-Propoxy, tert.-Butoxy, Benzyloxy, $C_1$-$C_4$-Alkyl, Methyl, Ethyl, Propyl, iso-Propyl, sek.-Butyl, tert.-Butyl, Neopentyl, Phenyl, Halogenphenyl, 4-Fluorphenyl, $C_1$-$C_4$-Alkylphenyl, 4-tert.-Butylphenyl, Hydroxymethyl, $C_1$-$C_4$-Alkoxymethyl, Methoxymethyl, Ethoxymethyl, tert.-Butoxymethyl, Acetoxymethyl, Pivaloyloxymethyl, Benzyloxymethyl, Benzyl, Hydroxyethyl, Methoxyethyl, tert.-Butoxyethyl, Acetoxyethyl, Benzoyloxyethyl
- X für Sauerstoff,
- Y für Wasserstoff oder Hydroxyl, Wasserstoff wird bevorzugt,
- $R^1$ beispielsweise für Wasserstoff, Methyl, Ethyl, 2,2-Dimethylpropyl, Cyclohexyl, Phenyl; Methyl wird bevorzugt.

Bevorzugte neue Verbindungen sind auch die Additionsprodukte aus Säuren und Verbindungen der Formel I in der der Rest A, der Index n und die Substituenten X, Y und $R^1$, $R^2$ und $R^3$ die oben genannten Bedeutungen haben.

Solche Säuren sind z.B. Mineralsäuren, wie beispielsweise Chlorwasserstoffsäure, Bromwasserstoffsäure, Phosphorsäure, Schwefelsäure, Salpetersäure, oder aber Carbonsäuren, wie Ameisensäure, Essigsäure, Oxalsäure, Malonsäure, Milchsäure, Äpfelsäure, Bernsteinsäure, Weinsäure, Zitronensäure, Salicylsäure, p-Toluolsulfonbsäure, Dodecylbenzolsulfonsäure oder aber auch allgemein Protonen-acide Verbindungen, z.B. Saccharin. Salze mit pflanzenverträglichen Säuren werden bevorzugt (pflanzenverträgliche Salze).

Die neuen Verbindungen der Formel I können bei der Herstellung als Gemische von Stereoisomeren (E/Z-Isomere, Diastereomere, Enantiomere) anfallen, die in üblicher Weise, z.B. durch Kristallisation oder Chromatographie, in die Einzelkomponenten getrennt werden können. Sowohl die einzelnen Isomeren als auch ihre Gemische können als Fungizide verwendet werden und werden von der Erfindung erfaßt.

Die neuen Verbindungen der Formel I lassen sich auf an sich bekannte Art und Weise (vgl. EP 281 842, 278 352) durch Umsetzung von substituierten Spiroheterocyclen der allgemeinen Formel V

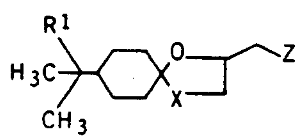

(V)

in welcher $R^1$ und X die oben angegebene Bedeutung haben und Z für eine elektronenziehende, nucleophil substituierbare Abgangsgrruppe steht, mit Heterocycloamin-Derivaten der allgemeinen Formel VI/VII/VIII

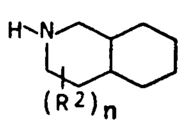

(VI)

,

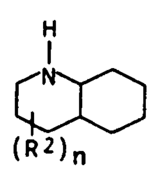

(VII)

oder

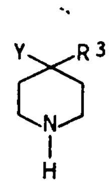

(VIII)

worin $R^2$, $R^3$, Y und n die oben angegebenen Bedeutungen haben, gegebenenfalls in Gegenwart eines Verdünnungsmittels und gegebenenfalls in Gegenwart einer Hilfsbase herstellen.

Die substituierten Spiroheterocyclen der allgemeinen Formel (V) sind bekannt oder lassen sich in an sich bekannter Weise herstellen (vgl. EP 281 842, 278 352 und darin zitierte Literatur). Die Perhydro(iso)-chinolin-Derivate der allgemeinen Formeln VI/VII sind teilweise bekannt oder lassen sich durch an sich bekannte Verfahren z.B. durch katalytische Hydrierung von (Iso)chinolin-Derivaten herstellen. Die Piperidin-Derivate der allgemeinen Formel VIII sind weitgehend bekannt oder lassen sich durch an sich bekannte Verfahren durch Hydrierung von Pyridin-Derivaten oder Derivatisierung von 4-Hydroxypiperidinen oder 4-Hydroxyalkylpiperidinen herstellen.

Herstellungsbeispiel 1

8-tert.-Butyl-2-[perhydroisochinolino-methyl]-1,4-dioxaspiro[4,5] decan (Verbindung Nr. 1.1)

10 g (34 mmol) 8-tert.-Butyl-2-brommethyl-1,4-dioxaspiro[4,5]decan, 5,2 g (37,4 mmol) Perhydroisochinolin (Isomerengemisch) und 4,7 g (34 mmol) Kaliumcarbonat wurden in 100 ml Acetonitril 16 Std. z. Rückfluß erhitzt. Das Lösungsmittel wurde i. Vak. abdestilliert, der erhaltene Rückstand mit verd. 5 %-iger NaOH und Methyl-tert.-butylether aufgenommen, die organische Phase wurde mit Wasser zweimal gewaschen, getrocknet ($Na_2SO_4$) und i. Vak. eingeengt. Fraktionierung des Rohprodukts i. Vak. ergab 4,9 g der Verbindung 1.1 (Sdp. 170 °C, 0,5 mbar).

Herstellung der Ausgangsverbindung

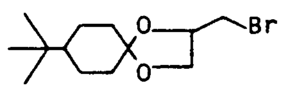

Zu 115,5 g (750 mmol) 4-tert.-Butylcyclohexanon in 700 ml abs. Dichlormethan wurden bei 5 °C 30,15 g (120 mmol) Zinn(IV)chlorid und anschließend innerhalb von 5 Std. 205,5 g (1,5 mol) Epibromhydrin

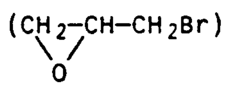

in 370 ml abs. Dichlormethan zugetropft. Die Mischung wurde über Nacht bei Raumtemperatur (20 °C) gerührt, unter Eiskühlung mit einer Lösung von 42 g KOH in 180 ml Wasser hydrolysiert und wie üblich aufgearbeitet.

Destillation des Rohprodukts (279 g) im Vakuum ergab 169 g (77,5 %) 8-tert.-Butyl-2-brommethyl-1,4-dioxaspiro[4,5]decan mit einem Siedepunkt von 110 °C/0,2 mbar.

Herstellungsbeispiel 2

8-tert.-Butyl-2-[(4-tert.-butylpiperidino)methyl]-1,4-dioxaspiro [4.5]decan (Verbindung Nr. 2.5)

17,4 g (123,6 mmol) 4-tert.-Butylpiperidin, 9,0 g (30,9 mmol) 8-tert.-Butyl-2-brommethyl-1,4-dioxaspiro-[4.5]decan (cis-trans-Gemisch) und 4,3 g (30,9 mmol) Kaliumcarbonat wurden in 100 ml abs. Dimethylformamid 8 Std. auf 150 °C erhitzt. Das Lösungsmittel wurde i.Vak. teilweise entfernt, der Rückstand mit verd. wäßriger Natronlauge/Methylenchlorid aufgenommen und wie üblich aufgearbeitet.

Das Rohprodukt wurde i. Vak. destilliert: 4,5 g, Sdp. 190 °C (0,3 Bär), (Diastereomerengemisch).

In entsprechender Weise lassen sich die anderen in den Tabellen 1 und 2 aufgeführten neuen Verbindungen herstellen.

Tabelle 1

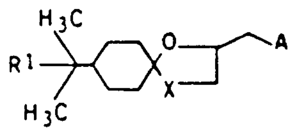

(I)

| Nr. | A | X | $R^1$ | Schmp./Spd. [°C], IR (Film) [$cm^{-1}$] |
|---|---|---|---|---|
| 1.1 | 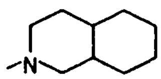 | O | $CH_3$ | 170°C/0,5 mbar |
| 1.3 | 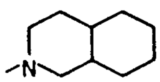 | O | $C_2H_5$ | |
| 1.4 | 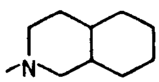 | O | Cyclohexyl | |
| 1.5 | 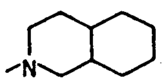 | O | Phenyl | |
| 1.6 | 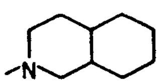 | O | 2-Methyl-propyl | |
| 1.7 | 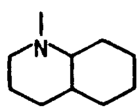 | O | $CH_3$ | |
| 1.8 | 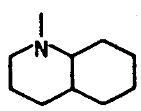 | O | $C_2H_5$ | |
| 1.10 | 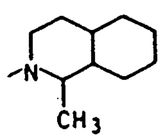 | O | $CH_3$ | |

Tabelle 1 (Fortsetzung)

| Nr. | A | X | $R^1$ | Schmp./Spd. [°C], IR (Film) [$cm^{-1}$] |
|---|---|---|---|---|
| 1.11 | 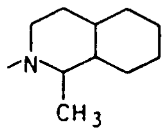 | O | $C_2H_5$ | |
| 1.12 | 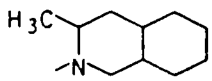 | O | $CH_3$ | |
| 1.13 | 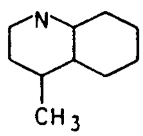 | O | $CH_3$ | |
| 1.14 | 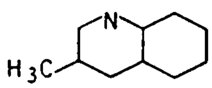 | O | $CH_3$ | |
| 1.15 | 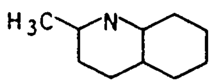 | O | $CH_3$ | |
| 1.16 | 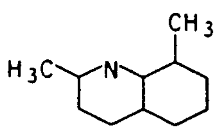 | O | $CH_3$ | |
| 1.17 | 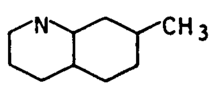 | O | $CH_3$ | |
| 1.18 | 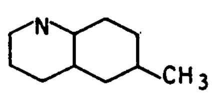 | O | $CH_3$ | |
| 1.19 | 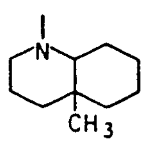 | O | $CH_3$ | |
| 1.20 | 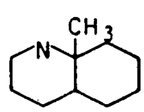 | O | $CH_3$ | |

Tabelle 1 (Fortsetzung)

| Nr. | A | X | $R^1$ | Schmp./Spd.[°C], IR (Film) [$cm^{-1}$] |
|---|---|---|---|---|
| 1.21 | 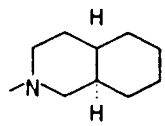 trans-Isomer | O | $CH_3$ | |
| 1.22 | 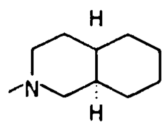 trans-Isomer | O | $CH_3$ | |
| 1.23 | 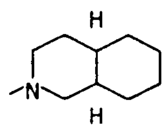 cis-Isomer | O | $CH_3$ | |
| 1.24 | 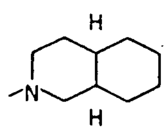 cis-Isomer | O | $CH_3$ | |
| 1.25 | 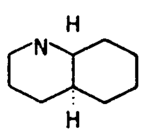 trans-Isomer | O | $CH_3$ | |
| 1.26 | 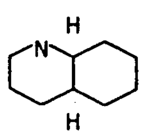 cis-Isomer | O | $CH_3$ | |
| 1.27 | 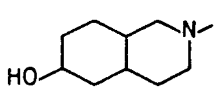 | O | $CH_3$ | |

Tabelle 1 (Fortsetzung)

| Nr. | A | X | $R^1$ | Schmp./Spd. [°C], IR (Film) [$cm^{-1}$] |
|---|---|---|---|---|
| 1.28 | 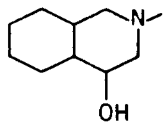 | O | $CH_3$ | |
| 1.29 | 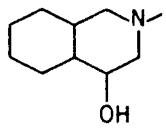 | O | $CH_3$ | |
| 1.30 | 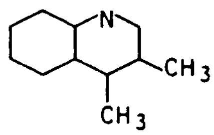 | O | $CH_3$ | |
| 1.31 | 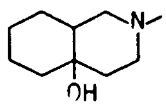 | O | $CH_3$ | |
| 1.32 | 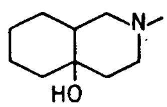 | O | $CH_3$ | |
| 1.33 | 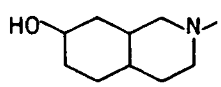 | O | $CH_3$ | |
| 1.34 | 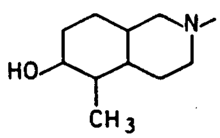 | O | $CH_3$ | |
| 1.35 | 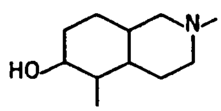 | O | $CH_3$ | |

Tabelle 2

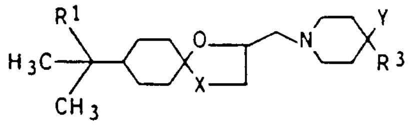

I

| Nr. | $R^1$ | X | $R^3$ | Y | Schmp., Sdp. [°C] IR (Film), [$cm^{-1}$] |
|---|---|---|---|---|---|
| 2.1 | $CH_3$ | O | $C_2H_5$ | H | |
| 2.2 | $CH_3$ | O | $n-C_3H_7$ | H | |
| 2.3 | $CH_3$ | O | $i-C_3H_7$ | H | |
| 2.4 | $CH_3$ | O | $sec-C_4H_9$ | H | |
| 2.5 | $CH_3$ | O | $tert.-C_4H_9$ | H | 190°/0,3 mbar |
| 2.6 | $CH_3$ | O | $-C(CH_3)_2-CH_2-CH_3$ | H | |
| 2.7 | $CH_3$ | O | $-C(CH_3)_2-CH_2-C(CH_3)_3$ | H | |
| 2.8 | $CH_3$ | O | $-OH$ | H | 160°/0,15 mbar |
| 2.9 | $CH_3$ | O | $O-\overset{\overset{O}{\|\|}}{C}-CH_3$ | H | |
| 2.10 | $CH_3$ | O | $O-\overset{\overset{O}{\|\|}}{C}-C_2H_5$ | H | |
| 2.11 | $CH_3$ | O | $O-\overset{\overset{O}{\|\|}}{C}-C_6H_5$ | H | |
| 2.12 | $CH_3$ | O | $O-\overset{\overset{O}{\|\|}}{C}-C(CH_3)_3$ | H | |
| 2.13 | $CH_3$ | O | $-OCH_3$ | H | |
| 2.14 | $CH_3$ | O | $-OC_2H_5$ | H | |
| 2.15 | $CH_3$ | O | $-O-CH(CH_3)_2$ | H | |
| 2.16 | $CH_3$ | O | $-O-C(CH_3)_3$ | H | |
| 2.17 | $CH_3$ | O | $-CH_2-OH$ | H | |
| 2.18 | $CH_3$ | O | $-CH_2O-CH_3$ | H | |
| 2.19 | $CH_3$ | O | $-CH_2-O-C_2H_5$ | H | |
| 2.20 | $CH_3$ | O | $-CH_2-O-\overset{\overset{O}{\|\|}}{C}-CH_3$ | H | |
| 2.21 | $CH_3$ | O | $-CH_2-CH_2-OH$ | H | |
| 2.22 | $CH_3$ | O | $-CH_2-CH_2-O\overset{\overset{O}{\|\|}}{C}CH_3$ | H | |
| 2.23 | $CH_3$ | O | $C_6H_5$ | H | |

Tabelle 2 (Fortsetzung)

| Nr. | $R^1$ | X | $R^3$ | Y | Schmp., Sdp. [°C] IR (Film), [$cm^{-1}$] |
|---|---|---|---|---|---|
| 2.24 | $CH_3$ | O | $C_6H_5$ | OH | 200°/0,1 mbar |
| 2.25 | $CH_3$ | O | $4-F-C_6H_5$ | OH | |
| 2.26 | $CH_3$ | O | $C_6H_5-CH_2-$ | H | Öl |
| 2.27 | $CH_3$ | O | $C_6H_5(CH_2)_2-$ | H | |
| 2.28 | $CH_3$ | O | $C_6H_5(CH_2)_3-$ | H | Öl; 2939,1465,1323, 1195,1103 $cm^{-1}$ |
| 2.29 | $CH_3$ | O | $4-t-C_4H_9-C_6H_4-$ | H | |

Tabelle 2 (Fortsetzung)

| Nr. | $R^1$ | X | $R^3$ | Y | Schmp., Sdp. [°C] IR (Film), [$cm^{-1}$] |
|---|---|---|---|---|---|
| 2.62 | H | O | $C_2H_5$ | H | |
| 2.63 | H | O | $i-C_3H_7$ | H | |
| 2.64 | H | O | $t-C_4H_9$ | H | |
| 2.65 | H | O | -OH | H | |
| 2.66 | H | O | $-O-CH_3$ | H | |
| 2.67 | H | O | $-O-\overset{O}{\overset{\|}{C}}CH_3$ | H | |
| 2.68 | H | O | $C_6H_5$ | H | |
| 2.69 | H | O | $C_6H_5$ | OH | |
| 2.70 | H | O | $C_6H_5CH_2$ | H | |
| 2.71 | $C_2H_5$ | O | $C_2H_5$ | H | |
| 2.72 | $C_2H_5$ | O | $n-C_3H_7$ | H | |
| 2.73 | $C_2H_5$ | O | $i-C_3H_7$ | H | |
| 2.74 | $C_2H_5$ | O | $sec-C_4H_9$ | H | |
| 2.75 | $C_2H_5$ | O | $tert.-C_4H_9$ | H | |
| 2.76 | $C_2H_5$ | O | $-C(CH_3)_2-CH_2-CH_3$ | H | |
| 2.77 | $C_2H_5$ | O | $-C(CH_3)_2-CH_2-C(CH_3)_3$ | H | |
| 2.78 | $C_2H_5$ | O | -OH | H | |
| 2.79 | $C_2H_5$ | O | $-O\overset{O}{\overset{\|}{C}}CH_3$ | H | |
| 2.80 | $C_2H_5$ | O | $-O-\overset{O}{\overset{\|}{C}}-C_2H_5$ | H | |

Tabelle 2 (Fortsetzung)

| Nr. | $R^1$ | X | $R^3$ | Y | Schmp., Sdp. [°C] IR (Film), [$cm^{-1}$] |
|---|---|---|---|---|---|
| 2.81 | $C_2H_5$ | O | $-O-\overset{\overset{O}{\|}}{C}-C(CH_3)_3$ | H | |
| 2.82 | $C_2H_5$ | O | $-O-\overset{\overset{O}{\|}}{C}-C_6H_5$ | H | |
| 2.83 | $C_2H_5$ | O | $-OCH_3$ | H | |
| 2.84 | $C_2H_5$ | O | $-OC_2H_5$ | H | |
| 2.85 | $C_2H_5$ | O | $-O-C(CH_3)_3$ | H | |
| 2.86 | $C_2H_5$ | O | $-CH_2-OH$ | H | |
| 2.87 | $C_2H_5$ | O | $-CH_2-O-CH_3$ | H | |
| 2.88 | $C_2H_5$ | O | $-CH_2-OC_2H_5$ | H | |
| 2.89 | $C_2H_5$ | O | $-CH_2-O-\overset{\overset{O}{\|}}{C}CH_3$ | H | |
| 2.90 | $C_2H_5$ | O | $-CH_2-CH_2-OH$ | H | |
| 2.91 | $C_2H_5$ | O | $-CH_2-CH_2-\overset{\overset{O}{\|}}{C}CH_3$ | H | |
| 2.92 | $C_2H_5$ | O | $C_6H_5$ | H | |
| 2.93 | $C_2H_5$ | O | 4-tert.-$C_4H_9$-$C_6H_4$ | H | |
| 2.94 | Cyclo-hexyl | O | tert.-$C_4H_9$ | H | |
| 2.95 | $C_6H_5$ | O | tert.-$C_4H_9$ | H | |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,

Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze. Es werden also die Pilze oder die von einer Pilzinfektion bedrohten Materialien, Pflanzen, Saatgüter oder der Erdboden mit einer fungizid wirksamen Menge der Wirkstoffe behandelt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz (Holzschutz) eingesetzt werden, z.B. gegen Paecilomyces variotii. Bei der Saatgutbehandlung werden im allgemeinen Wirkstoffmengen von 0,001 bis 50 g, vorzugsweise 0,01 bis 10 g, je Kilogramm Saatgut benötigt.

Die Mittel bzw. die daraus hergestellten gebrauchsfertigen Zübereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1.1 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 2.5 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1.1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 2.5 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1.1 werden mit 3 Gew.-Teilen das Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 2.5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1.1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 2.5 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde die Verbindung 8-tertiär-Butyl-2-(piperidino-N-methyl)-1,4-dioxaspiro[4.5]-decan (A) - bekannt aus EP 281 842 - benutzt.

Anwendungsbeispiel 1

Wirksamkeit gegen Weizenbraunrost

Blätter von in Töpfen gewachsenen Weizensämlingen der Sorte "Kanzler" wurden mit Sporen des Braunrostes (Puccinia recondita) bestäubt. Danach wurden die Töpfe für 24 Stunden bei 20 bis 22°C in eine Kammer mit hoher Luftfeuchtigkeit (90 bis 95 %) gestellt. Während dieser Zeit keimten die Sporen aus und die Keimschläuche drangen in das Blattgewebe ein. Die infizierten Pflanzen wurden anschließend mit wäßrigen Spritzbrühen, die 80 % Wirkstoff und 20 % Emulgiermittel in der Trockensubstanz enthielten, tropfnaß gespritzt. Nach dem Antrocknen des Spritzbelages wurden die Versuchspflanzen im Gewächshaus bei Temperaturen zwischen 20 und 22°C und 65 bis 70 % relativer Luftfeuchte aufgestellt. Nach 8 Tagen wurde das Ausmaß der Rostpilzentwicklung auf den Blättern ermittelt.

Das Ergebnis zeigt, daß die Wirkstoffe Nr. 1.1 und 2.5 bei der Anwendung als 0,006 %ige (Gew.%) Spritzbrühe eine bessere fungizide Wirkung zeigten (100 %) als der bekannte Vergleichswirkstoff A (75 %).

Anwendungsbeispiel 2

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus besprüht. Nach etwa 20 Stunden wurden die Versuchspflanzen mit wäßrigen Spritzbrühe, die 80 % Wirkstoff und 20 % Emulgiermittel in der Trockensubstanz enthielt bis zur Tropfnässe besprüht. Nach dem Antrocknen des Spritzbelages wurden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wurde das Ausmaß der Pilzentwicklung nach 21 Tagen ermittelt.

Das Ergebnis zeigt, daß die Wirkstoffe Nr. 1.1 und 2.5 bei der Anwendung als 0,025 %ige Spritzbrühe eine bessere fungizide Wirkung zeigten (100 %) als der bekannte Vergleichswirkstoff A (40 %).

**Patentansprüche**

**1.** N-Heterocyclylomethyl-spiroheterocyclen der allgemeinen Formel I,

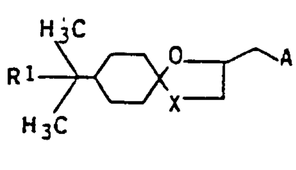

(I)

A für die Reste II, III oder IV

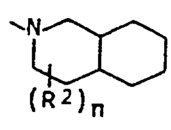
(II)

,

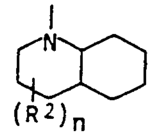
(III)

oder

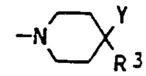
(IV)

$R^2$ für $CH_3$, OH, $OCH_3$,
n für die Zahlen 0 bis 3,
$R^3$ für Hydroxy, $C_1$-$C_4$-Acyloxy, $C_1$-$C_8$-Alkoxy, Benzoyloxy oder Benzyloxy steht, welche ein- bis dreifach durch Halogen oder $C_1$-$C_4$-Alkyl substituierte sein können,
für $C_1$-$C_8$-Alkyl oder $C_3$-$C_4$-Alkenyl steht, welche durch Hydroxy, Phenyl, $C_1$-$C_4$-Acyloxy, Benzoyloxy, $C_1$-$C_8$-Alkoxy oder Benzyloxy substituiert sein können, oder
für Phenyl steht, welches durch ein bis drei Halogen- oder $C_1$-$C_4$-Alkylreste subsituiert sein kann;
X für Sauerstoff steht,
Y für Wasserstoff oder Hydroxyl steht,
$R^1$ für Wasserstoff oder $C_1$-$C_6$-Alkyl steht oder für Cyclohexyl oder Phenyl steht, welche ein- bis drei fach durch Halogen oder $C_1$-$C_4$-Alkyl substituiert sein können,

sowie deren Säureadditionssalze.

**2.** N-Heterocyclylomethylspiroheterocyclen gemäß Anspruch 1, in denen A für den Rest II steht.

**3.** N-Heterocyclylomethylspiroheterocyclen gemäß Anspruch 1, in denen A für den Rest III steht.

**4.** N-Heterocyclylomethylspiroheterocyclen gemäß Anspruch 1, in denen A für den Rest IV steht.

**5.** 8-(tert.-Butyl)-2-(isochinolinomethyl)-1,4-dioxaspiro-[4,5]-decan.

**6.** 8-(Prop-2-yl)-2-(isochinolinomethyl)-1,4-dioxaspiro-[4,5]-decan.

**7.** 8-(2-Methyl-but-2-yl)-2-[(4-tert.-butylpiperidino)methyl]-1,4-dioxaspiro-[4,5]-decan.

**8.** Fungizides Mittel, enthaltend einen festen oder flüssigen Trägerstoff und eine fungizid wirksame Menge einer Verbindung der Formel I gemäß Anspruch 1, oder dessen Säureadditionssalz.

**9.** Verfahren zur Herstellung von Fungiziden, dadurch gekennzeichnet, daß man eine Verbindung der Formel I gemäß Anspruch 1 mit einem festen oder flüssigen Trägerstoff mischt.

**10.** Verfahren zur Bekämpfung von Pilzen, dadurch gekennzeichnet, daß man eine fungizid wirksame Menge einer Verbindung der Formel I gemäß Anspruch 1 oder deren Säureadditionssalz auf die Pilze oder auf durch Pilzbefall bedrohte Materialien, Flächen, Pflanzen oder Saatgüter oder den Boden einwirken läßt, mit Ausnahme von Verfahren zur Bekämpfung von Pilzen am menschlichen und tierischen Körper.

**11.** Verfahren zur Herstellung der Verbidnungen I, dadurch gekennzeichnet, daß man einen Spiroheterocyclus der Formel V,

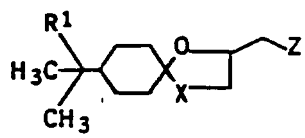
(V),

in welcher Z für eine elektronenziehende, nukleophil substituierbare Abgangsgruppe steht, mit einem Heterocycloamidderivat der Formel VI, VII oder VIII

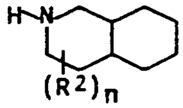
(VI)

,

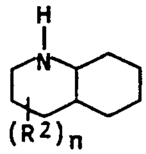
(VII)

oder

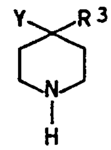
(VIII)

umsetzt.

## Claims

**1.** An N-heterocyclomethylspiroheterocycle of the general formula I

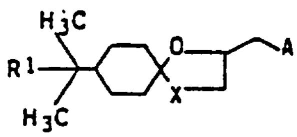
(I)

where

A is a radical II, III or IV

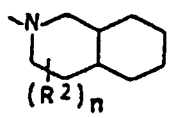
(II)

,

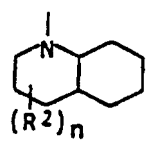
(III)

or

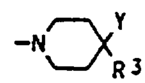
(IV)

$R^2$ is $CH_3$, OH or $OCH_3$,

n is from 0 to 3,

$R^3$ is hydroxyl, $C_1$-$C_4$-acyloxy, $C_1$-$C_8$-alkoxy, benzoyloxy or benzyloxy which can each be monosubstituted to trisubstituted by halogen or $C_1$-$C_4$-alkyl, $C_1$-$C_8$-alkyl or $C_3$- or $C_4$-alkenyl which can each be substituted by hydroxyl, phenyl, $C_1$-$C_4$-acyloxy, benzoyloxy, $C_1$-$C_8$-alkoxy or benzyloxy, or
phenyl which can be substituted by one to three halogen or $C_1$-$C_4$-alkyl radicals,

X is oxygen,

Y is hydrogen or hydroxyl, and

$R^1$ is hydrogen or $C_1$-$C_6$-alkyl or cyclohexyl or phenyl which can each be monosubstituted to trisubstituted by halogen or $C_1$-$C_4$-alkyl, or an addition salt thereof with an acid.

**2.** An N-heterocyclomethylspiroheterocycle as claimed in claim 1, wherein A is the radical II.

**3.** An N-heterocyclomethylspiroheterocycle as claimed in claim 1, wherein A is the radical III.

**4.** An N-heterocyclomethylspiroheterocycle as claimed in claim 1, wherein A is the radical IV.

**5.** 8-(tert-Butyl)-2-(isoquinolinomethyl)-1,4-dioxaspiro[4,5]decane.

**6.** 8-(Prop-2-yl)-2-(isoquinolinomethyl)-1,4-dioxaspiro[4,5]decane.

**7.** 8-(2-Methylbut-2-yl)-2-[(4-tert-butylpiperidino)methyl]-1,4-dioxaspiro[4,5]decane.

**8.** A fungicide containing a solid or liquid carrier and a fungicidal amount of a compound of the formula I as claimed in claim 1, or an addition salt thereof with an acid.

**9.** A process for the preparation of fungicides, which comprises mixing a compound of the formula I as claimed in claim 1 with a solid or liquid carrier.

**10.** A method for controlling fungi, which comprises allowing a fungicidal amount of a compound of the formula I as claimed in claim 1, or an addition salt thereof with an acid, to act on the fungi or on materials, areas, plants or seeds threatened by fungal attack, or on the soil, with the exception of methods for controlling fungi in humans and animals.

**11.** A process for the preparation of a compound I, which comprises reacting a spiroheterocycle of the formula V

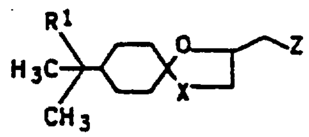

(V),

where Z is an electron-attracting, nucleophilically substitutable leaving group, with a heterocycloamine derivative of the formula VI, VII or VIII

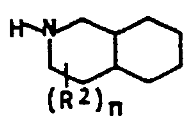

(VI)

,

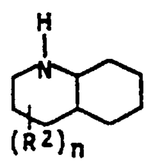

(VII)

or

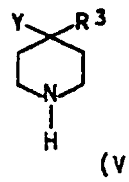

(VIII).

## Revendications

**1.** N-hétérocyclylomethyl-spirohétérocycles de la formule I

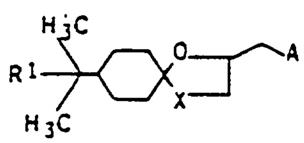

(I)

dans laquelle

A est mis pour les restes II, III ou IV

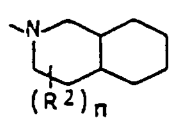

(II)

,

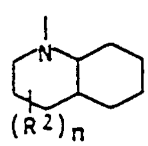

(III)

ou

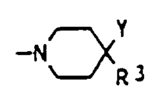

(IV)

$R^2$ pour $CH_3$, OH, $OCH_3$,

n pour les nombres 0 à 3,

$R^3$ pour hydroxy, acyloxy en C1-C4, alcoxy en C1-C6, benzoyloxy ou benzyloxy, qui peuvent être substitués une à trois fois par halogène ou alkyle en C1-C4,

pour alkyle en C1-C8 ou alcényle en C3-C4, qui peuvent être substitués par hydroxy, phényle, acyloxy en C1-C4, benzoyloxy, alcoxy en C1-C8 ou benzoyloxy, ou

pour phényle qui peut être substitué par un à trois restes halogène ou alkyle en C1-C4,

X pour oxygène,

Y pour hydrogène ou hydroxyle,

$R^1$ pour hydrogène ou alkyle en C1-C6 ou pour cyclohexyle ou phényle, qui peuvent être substitués une à trois fois par halogène ou alkyle en C1-C4,

ainsi que leurs sels d'addition d'acide.

**2.** N-hétérocyclylométhyl-spirohétérocycles selon la revendication 1, dans lequel A est mis pour le reste II.

**3.** N-hétérocyclylométhyl-spirohétérocycles selon la revendication 1, dans lequel A est mis pour le reste III.

**4.** N-hétérocyclylométhyl-spirohétérocycles selon la revendication 1, dans lequel A est mis pour le reste IV.

**5.** 8-(tert-butyl)-2-isoquinoléinométhyl)-1,4-dioxaspiro-[4,5]-décane.

**6.** 8-(prop-2-yl)-2-(isoquinoélinométhyl)-1,4-dioxaspiro-[4,5]-décane.

**7.** 8-(2-méthyl-but-2-yl)-2-[(4-tert-butylpipéridino)méthyl]-1,4-dioxaspiro-[4,5]-décane.

**8.** Fongicide contenant un support solide ou liquide et une quantité à action fongicide d'un composé de formule I selon la revendication 1 ou ses sels d'addition d'acide.

**9.** Procédé de préparation de fongicides, caractérisé par le fait qu'on mélange un composé de formule I selon la revendication 1 avec un support solide ou liquide.

**10.** Procédé pour lutter contre les champignons, caractérisé par le fait que l'on fait agir une quantité à action fongicide d'un composé de formule I selon la revendication 1 ou ses sels d'addition d'acide sur les champignons ou sur les matériaux menacés d'une attaque par les champignons, surfaces, plantes ou semences ou les sols menacés d'une attaque par les champignons, en excluant les procédés de lutte contre les champignons sur le corps humain ou animal.

**11.** Procédé de préparation des composés I, caractérisé par le fait que l'on fait réagir un spirohétérocycle de la formule V

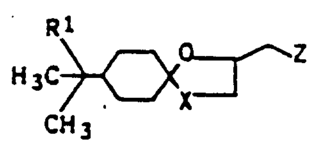

(V),

dans laquelle Z est mis pour un groupe éliminable attirant les électrons et substituable par action nucléophile avec un dérivé d'hétérocyloamide de formule VI, VII ou VIII

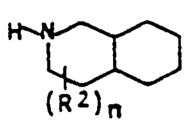

(VI)

,

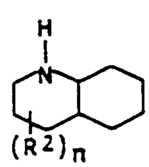

(VII)

ou

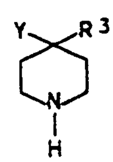

(VIII)